# EUROPÄISCHE PATENTSCHRIFT

(11) **EP 3 784 157 B1**
(45) Veröffentlichungstag und Bekanntmachung des Hinweises auf die Patenterteilung: **03.07.2024**
(21) Anmeldenummer: 19726582.0
(22) Anmeldetag: 04.05.2019
(51) Int. Cl.: A61B 18/12, A61B 18/14, A61B 18/00, A61B 17/00

(54) **ELEKTROCHIRURGISCHES SYSTEM, SOWIE ULTRASCHALLGENERATOR DAFÜR**
ELECTROSURGICAL SYSTEM, AND ULTRASOUND GENERATOR FOR SAME
SYSTÈME ÉLECTROCHIRURGICAL, AINSI QUE GÉNÉRATEUR D'ULTRASONS POUR CELUI-CI

(30) Priorität: 04.06.2018 DE 102018113261
(43) Veröffentlichungstag der Anmeldung: 03.03.2021
(73) Patentinhaber: OLYMPUS WINTER & IBE GMBH, 22045 Hamburg (DE)
(72) Erfinder: FAEHSING, Thomas, 12305 Berlin (DE)
(74) Vertreter: Noack, Andreas
(86) Internationale Anmeldenummer: PCT/EP2019/061469
(87) Internationale Veröffentlichungsnummer: WO 2019/233683

(56) Entgegenhaltungen:
- EP-A1- 1 199 044
- EP-A2- 1 199 047
- US-A1- 2002 062 132

## Beschreibung

Die Erfindung betrifft ein Elektrochirurgisches System mit einem Ultraschallgenerator, der zur Abgabe eines hochfrequenten elektrischen Signals eingerichtet ist, und einem Ultraschallinstrument, welches einen Ultraschall-Transducer umfasst, der zum Umsetzen des hochfrequenten elektrischen Signals in eine Ultraschallschwingung eingerichtet ist, wobei der Ultraschallgenerator ferner eingerichtet ist, eine Resonanzfrequenz des Ultraschall-Transducers zu bestimmen und eine Frequenz des hochfrequenten elektrischen Signals an diese Resonanzfrequenz anzupassen, und wobei der Ultraschallgenerator weiter eingerichtet ist, eine Phasenlage zwischen dem Strom und der Spannung des hochfrequenten elektrischen Signals zu ermitteln und anhand der ermittelten Phasenlage zu bestimmen, ob die Frequenz des hochfrequenten elektrischen Signals der Resonanzfrequenz des Ultraschall-Transducers entspricht.

Weiterhin betrifft die Erfindung einen Ultraschallgenerator.

In der modernen Elektrochirurgie werden neben reinen elektrochirurgischen Verfahren, in denen ein chirurgischer Effekt ausschließlich durch elektrische Ströme erreicht wird, auch Verfahren und Instrumente verwendet, bei denen ein hochfrequentes elektrisches Signal mittels eines Ultraschall-Transducers in eine Ultraschallschwingung umgesetzt wird, welche dann einen chirurgischen Effekt bewirkt. Dabei können ein durch Stromfluss bewirkter chirurgischer Effekt und ein durch Ultraschall bewirkter chirurgischer Effekt kombiniert werden.

Hierzu weisen entsprechende elektrochirurgische Systeme einen Ultraschallgenerator auf. Dieser Ultraschallgenerator erzeugt ein hochfrequentes elektrisches Signal, welches einem Ultraschallinstrument zugeführt wird. Das Ultraschallinstrument umfasst einen Ultraschall-Transducer, bei dem es sich in der Regel um ein piezoelektrisches Element handelt, welches bei Anliegen des hochfrequenten elektrischen Signals eine Ultraschallschwingung erzeugt. Die Ultraschallschwingung wird im Ultraschallinstrument auf ein Arbeitselement übertragen, welches bei Kontakt mit biologischem Gewebe einen chirurgischen Effekt bewirkt. Das Arbeitselement wird auch als Sonotrode bezeichnet.

Um einen wirksamen chirurgischen Effekt zu erzielen, wird der Ultraschall-Transducer in seiner Resonanzfrequenz betrieben. Diese Resonanzfrequenz hängt zum einen vom Typ des Ultraschallinstruments ab, zum anderen aber auch von Fertigungstoleranzen der Komponenten des Ultraschallinstruments und von externen Faktoren, wie z.B. der mechanischen Belastung während der Verwendung des Ultraschallinstruments. Die Resonanzfrequenz liegt in der Regel zwischen 10kHz und 100 kHz, beispielsweise bei 50kHz.

Um den Ultraschall-Transducer in seiner Resonanzfrequenz ansteuern zu können ist der Ultraschallgenerator eingerichtet, diese Resonanzfrequenz zu bestimmen. Dazu führt der Ultraschallgenerator bei unbelastetem Ultraschallinstrument einen sogenannten Scan-Vorgang durch, bei dem der Ultraschall-Transducer mit unterschiedlichen Frequenzen angesteuert wird. Da die Stärke der mechanischen Ultraschallschwingung nicht direkt gemessen werden kann, misst der Ultraschallgenerator ständig den Strom und die Spannung des hochfrequenten elektrischen Signals, und ermittelt die Phasenlage zwischen den beiden Größen. Aus der Phasenlage lässt sich ermitteln, ob die Frequenz des hochfrequenten elektrischen Signals der Resonanzfrequenz des Ultraschall-Transducers entspricht. Bei der Resonanzfrequenz sind Strom und Spannung des hochfrequenten Signals in Phase.

Während der Anwendung ändert sich durch wechselnde mechanische Belastung des Ultraschallinstruments die Resonanzfrequenz. Um die Frequenz des hochfrequenten elektrischen Signals bei Änderungen der Resonanzfrequenz nachzuführen, misst der Ultraschallgenerator weiter die Phasenlage von Strom und Spannung und regelt die Frequenz so, dass die im Scan-Vorgang ermittelte Phasenlage erhalten bleibt.

Die EP 1 199 044 A1 beschreibt ein elektrochirurgisches System, in welchem ein Ultraschall-Transducer mittels eines hochfrequenten elektrischen Signals in eine Ultraschallschwingung versetzt wird. Zur Steuerung einer Amplitude der Ultraschallschwingung werden die Impedanz des Ultraschall-Transducers und die Phase zwischen Strom und Spannung des hochfrequenten elektrischen Signals ermittelt.

Bei der Messung der Phasenlage zwischen Strom und Spannung des elektrischen Signals treten systematische Messfehler auf, welche durch Frequenzgänge und Toleranzen eingesetzter elektronischer Komponenten verursacht werden. Es kann daher vorkommen, dass der Ultraschall-Transducer nicht genau in seiner Resonanzfrequenz betrieben wird. Dies führt zu einem reduzierten Wirkungsgrad des Ultraschallinstruments, wobei der Ultraschallgenerator und das Ultraschallinstrument durch anfallende Verlustleistung belastet und eventuell beschädigt werden können.

Es besteht daher die Aufgabe der Erfindung darin, ein elektrochirurgisches System bereitzustellen, welches hinsichtlich der beschriebenen Problematik verbessert ist.

Diese Aufgabe wird gemäß eines ersten Aspekts der Erfindung gelöst durch ein Elektrochirurgisches System mit einem Ultraschallgenerator, der zur Abgabe eines hochfrequenten elektrischen Signals eingerichtet ist, und einem Ultraschallinstrument, welches einen Ultraschall-Transducer umfasst, der zum Umsetzen des hochfrequenten elektrischen Signals in eine Ultraschallschwingung eingerichtet ist, wobei der Ultraschallgenerator ferner eingerichtet ist, eine Resonanzfrequenz des Ultraschall-Transducers zu bestimmen und eine Frequenz des hochfrequenten elektrischen Signals an diese Resonanzfrequenz anzupassen, und wobei der Ultraschallgenerator weiter eingerichtet ist, eine Phasenlage zwischen dem Strom und der Spannung des hochfrequenten elektrischen Signals zu ermitteln und anhand der ermittelten Phasenlage zu bestimmen, ob die Frequenz des hochfrequenten elektrischen Signals der Resonanzfrequenz des Ultraschall-Transducers entspricht, welches dadurch weitergebildet ist, dass der Ultraschallgenerator eingerichtet ist, bei der Bestimmung der Phasenlage Korrekturwerte zu berücksichtigen, die in einem Speicher des Ultraschallgenerators und/oder des Ultraschallinstruments hinterlegt sind oder hinterlegbar sind.

Bei den Korrekturwerten kann es sich beispielsweise um feste Beträge handeln, welche zu einer vom Ultraschallgenerator gemessene Phasenlage addiert werden, um eine korrigierte Phasenlage zu erhalten, welche zur Frequenzregelung verwendet wird.

Die Korrekturwerte können auch Parameter von Korrekturfunktionen beinhalten, welche eine Phasenkorrektur in Abhängigkeit verschiedener Betriebsparameter des Ultraschallgenerators abbilden, z.B. in Abhängigkeit von der Betriebsfrequenz oder der Ausgangsleistung des Ultraschallgenerators.

In einer möglichen Ausführung der Erfindung kann der Ultraschallgenerator Sensoren zur Messung des Stroms und/oder der Spannung des hochfrequenten Signals aufweisen, wobei ein Frequenz- und/oder Phasengang der Sensoren die Ermittlung der Phasenlage zwischen dem Strom und der Spannung des hochfrequenten elektrischen Signals beeinflusst, und wobei die Korrekturwerte erste Korrekturwerte umfassen, durch deren Berücksichtigung diese Beeinflussung ganz oder teilweise kompensiert wird.

Die Messchaltungen können z.B. analoge Filter, Analog-Digital-Wandler, und/oder digitale Filter umfassen. Eine durch solche Elemente verursache Phasenverschiebung der Messwerte kann recht genau theoretisch bestimmt oder während der Kalibrierung eines Ultraschallgenerators messtechnisch ermittelt werden. Die ermittelten Werte können als erste Korrekturwerte in einem Speicher des Ultraschallgenerators abgelegt werden und stehen so jederzeit zur Verfügung, um eine korrigierte Bestimmung der Phasenlage zwischen Strom und Spannung des hochfrequenten elektrischen Signals zu ermöglichen.

Gemäß einer weiteren vorteilhaften Ausführungsform der Erfindung stellt der Ultraschall-Transducer eine kapazitive Last dar, und der Ultraschallgenerator umfasst eine Induktivität, um durch die kapazitive Last verursachte Blindströme zu unterdrücken, wobei eine Fehlanpassung zwischen der kapazitiven Last und der Induktivität die Ermittlung der Phasenlage zwischen dem Strom und der Spannung des hochfrequenten elektrischen Signals beeinflusst, und wobei die Korrekturwerte zweite Korrekturwerte umfassen, durch deren Berücksichtigung diese Beeinflussung ganz oder teilweise kompensiert wird.

Ultraschall-Transducer weisen in der Regel ein kapazitives Verhalten auf, welches im Wesentlichen durch eine vom mechanischen Verhalten des Ultraschall-Transducers unabhängige Parallelkapazität geprägt ist. Um die durch diese Kapazität verursachten Blindströme zu reduzieren ist am Ausgang vieler Ultraschallgeneratoren eine Induktivität parallel zum Ultraschall-Transducer geschaltet. Da jedoch die Parallelkapazität des Ultraschall-Transducers Fertigungsschwankungen unterworfen ist, kann es zu Fehlanpassungen zwischen dem Ultraschall-Transducer und dem Ultraschallgenerator kommen, welche die Phasenlage zwischen dem Strom und der Spannung des hochfrequenten elektrischen Signals beeinflussen.

Um dies zu kompensieren kann die genaue Parallelkapazität eines Ultraschall-Transducers nach dessen Fertigung bestimmt und als zweiter Korrekturwert in dem Speicher abgelegt werden. Diese kann dann durch den Ultraschallgenerator bei der Bestimmung der Phasenlage berücksichtigt werden.

In einer bevorzugten Ausführungsform der Erfindung können die zweiten Korrekturwerte in dem Ultraschallinstrument gespeichert sein, und der Ultraschallgenerator kann eingerichtet sein, die zweiten Korrekturwerte bei einer Verbindung des Ultraschallinstruments mit dem Ultraschallgenerator auszulesen.

Es ist dadurch möglich, die zweiten Korrekturwerte zu ermitteln und zu speichern, ohne dass feststeht, mit welchem Ultraschallgenerator das jeweilige Ultraschallinstrument verwendet werden soll. Das elektrochirurgische System wird dadurch sehr flexibel.

Es ist auch denkbar, bei der ersten Verbindung eines Ultraschallinstruments mit einem Ultraschallgenerator die sich ergebende Phasenbeeinflussung zu messen und als zweiten Korrekturwert in einem Speicherelement des Ultraschallgenerators oder des Ultraschallinstruments zu speichern. Der Ultraschallgenerator kann dann bei späteren Verwendungen des selben Ultraschallinstruments diese Phasenbeeinflussung aus dem Speicher lesen und entsprechend berücksichtigen.

In einer vorteilhaften Ausführung der Erfindung kann ein Ultraschallgenerator mehrere Induktivitäten umfassen, welche in Abhängigkeit von einem Typ eines angeschlossenen Ultraschallinstruments wahlweise zu- oder abgeschaltet werden können. Auf diese Weise kann ein Ultraschallgenerator mit verschiedenen Typen von Ultraschallinstrumenten optimal angepasst betrieben werden.

Die Zu- oder Abschaltung von Induktivitäten kann dabei vorzugsweise auf Basis einer automatischen Instrumentenerkennung erfolgen. Dazu können beispielsweise Identifikationsdaten in einem Speicherelement des Ultraschallinstruments abgelegt sein, anhand derer der Ultraschallgenerator erkennt, um welchen Instrumententyp es sich handelt. Die ersten und/oder zweiten Korrekturwerte sind vorzugsweise durch eine Kalibriermessung bestimmt oder bestimmbar.

Nach einer besonderen Ausführungsform der Erfindung ist der Ultraschallgenerator eingerichtet, Strom und Spannung des hochfrequenten elektrischen Signals zeitdiskret abzutasten und aus dem Verlauf der Abtastwerte die Phasenlage zu bestimmen, wobei zur Berücksichtigung der Korrekturwerte die Abtastwerte des Stroms gegenüber den Abtastwerten der Spannung um eine aus den Korrekturwerten abgeleitete Zeitdifferenz verzögert werden, oder die Abtastwerte der Spannung gegenüber den Abtastwerten des Stroms um eine aus den Korrekturwerten abgeleitete Zeitdifferenz verzögert werden.

Bei einer entsprechenden zeitdiskreten Verarbeitung der Abtastwerte von Strom und Spannung ist die Berücksichtigung der Korrekturwerte besonders einfach umzusetzen. Die Verarbeitung der Abtastwerte erfolgt dabei bevorzugt im Rahmen eines Programmablaufs in einem Prozessor, der Bestandteil des Ultraschallgenerators ist. Dabei können die zu verzögernden Abtastwerte durch eine First-In-First-Out (FIFO) Datenstruktur geführt werden, wobei sich die Zeitdifferenz aus dem Produkt der Anzahl der Speicherstellen der FIFO Datenstruktur und der Taktrate der Programmausführung ergibt. Als FIFO-Datenstruktur kommt beispielsweise ein Schieberegister in Frage.

Die Aufgabe wird gemäß weiterer Aspekte der Erfindung gelöst durch einen Ultraschallgenerator eines elektrochirurgischen Systems gemäß den obigen Ausführungen. Bezüglich der hierdurch erreichten Wirkungen und Vorteile wird ausdrücklich auf das oben gesagte verwiesen.

Die Erfindung wird nachfolgend anhand einiger beispielhafter Darstellungen näher erläutert. Die dargestellten Ausführungsbeispiele dienen hierbei lediglich zum besseren Verständnis der Erfindung, ohne diese einzuschränken.

Es zeigen:
Fig. 1: ein elektrochirurgisches System,
Fig. 2: den Aufbau eines Ultraschallinstruments,
Fig. 3: Phasengang eines Ultraschall-Transducers,
Fig. 4: schematischer Aufbau eines Utraschallgenerators,
Fig. 5: ein möglicher Ablauf der Phasenkorrektur.

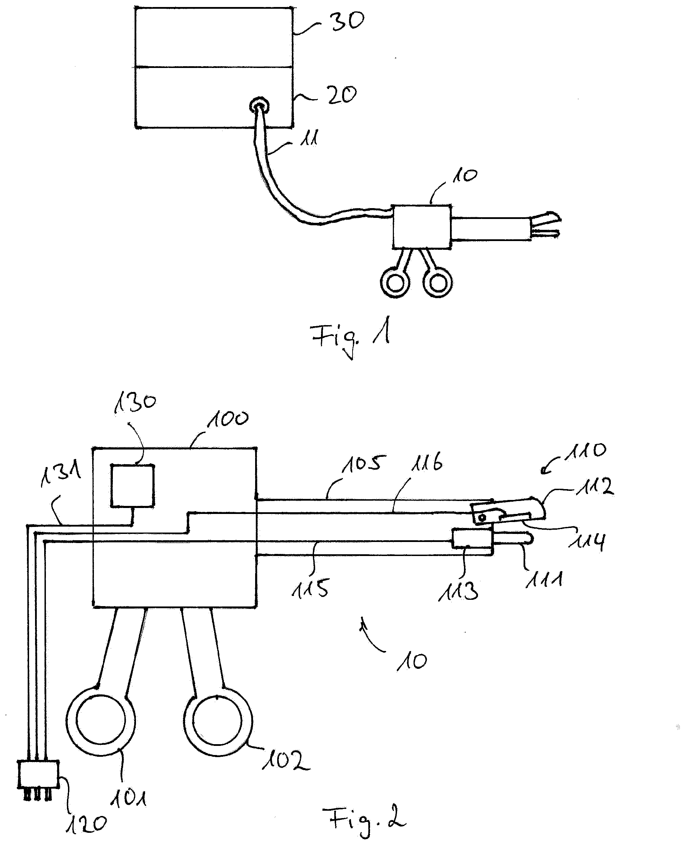

Figur 1 zeigt ein elektrochirurgisches System mit einem Ultraschallinstrument 10, einem Ultraschallgenerator 20, sowie einem Hochfrequenzgenerator 30. Das Ultraschallinstrument 10 ist über ein Kabel 11 mit dem Ultraschallgenerator 20 verbunden.

Das Ultraschallinstrument 10 kann beispielsweise eine kombinierte Hochfrequenz- und Ultraschallzange sein, wie sie beispielsweise unter dem Namen THUNDERBEAT von der Olympus Corporation vertrieben wird.

Im Betrieb erzeugt der Ultraschallgenerator 20 ein erstes hochfrequentes elektrisches Signal, welches über das Kabel 11 an das Ultraschallinstrument 10 übertragen wird, und dort von einem nicht dargestellten Ultraschall-Transducer in eine Ultraschall-Schwingung umgesetzt wird. Die Ultraschallschwingung wird in eine nicht dargestellte Sonotrode eingekoppelt, welche direkt oder indirekt mit zu behandelndem Gewebe in Kontakt gebracht werden kann.

Der Hochfrequenzgenerator 30 erzeugt im Betrieb ein zweites hochfrequentes elektrisches Signal, welches über eine interne Verbindung an den Ultraschallgenerator 20 und von dort ebenfalls über das Kabel 11 an das Ultraschallinstrument 10 übertragen wird. Im Ultraschallinstrument 10 wird das zweite hochfrequente elektrische Signal an eine oder mehrere Elektroden geführt, welche direkt oder indirekt mit zu behandelndem Gewebe in Kontakt gebracht werden können.

In Figur 2 ist der Aufbau des Ultraschallinstruments 10 näher dargestellt, wobei die Darstellung nicht maßstabsgetreu und stark vereinfacht ist.

Das Ultraschallinstrument 10 besteht aus einem Hauptkörper 100 mit Griffhebeln 101,102. An den Hauptkörper 100 schließt ein Schaft 105 an, an dessen distalem Ende ein Zangenmaul 110 angeordnet ist.

Das Zangenmaul 110 umfasst hier eine feststehende Branche, welche durch eine Sonotrode 111 gebildet ist, und eine bewegliche Branche 112. Die Sonotrode 111 ist mit einem Ultraschall-Transducer 113 gekoppelt. An der beweglichen Branche 112 ist eine Elektrode 114 angeordnet.

Die bewegliche Branche 112 kann durch Betätigen eines der Griffhebel 101,102 in Richtung der Sonotrode 111 bewegt werden, so dass sich das Zangenmaul 110 schließt. Ein nicht dargestellter Abschnitt von menschlichem oder tierischem Gewebe, der in dem geschlossenen Zangenmaul 110 eingeklemmt ist, kann dann durch Aktivieren der Sonotrode 111 und/oder der Elektrode 114 behandelt werden.

Zur Aktivierung der Sonotrode 111 wird ein erstes hochfrequentes elektrisches Signal von dem Ultraschallgenerator 20 dem Ultraschall-Transducer 113 zugeführt. Dieser setzt das Signal in eine Ultraschallschwingung um und überträgt diese an die Sonotrode 111. Die mechanische Bewegung der Sonotrode 111, die in engem Kontakt mit dem zu behandelnden Gewebe steht, bewirkt dann einen chirurgischen Effekt in dem Gewebe, welcher je nach Ausführung der Sonotrode und gewünschtem Ergebnis variieren kann.

Zur Aktivierung der Elektrode 114 wird dieser ein zweites hochfrequentes elektrisches Signal von dem elektrochirurgischen Generator 30 zugeführt.

Die Zuführung der elektrischen Signale erfolgt über Leitungen 115,116. Die Leitungen 115,116 enden in einem Stecker 120, der mit dem Ultraschallgenerator 20 verbunden werden kann.

Das Ultraschallinstrument 10 umfasst weiterhin ein Speicherelement 130, dessen Funktion später erläutert wird. Das Speicherelement 130 ist über eine Leitung 131 ebenfalls mit dem Stecker 120 verbunden.

Um einen optimalen chirurgischen Effekt zu erzielen ist es wünschenswert, dass der Ultraschallgenerator 20 den Ultraschall-Transducer 113 in seiner mechanischen Resonanzfrequenz ansteuert. Diese mechanische Resonanzfrequenz hängt jedoch von diversen Parametern ab, beispielsweise Fertigungstoleranzen des Ultraschall-Transducers 113, aber auch Art und Menge des im Zangenmaul 110 gegriffenen Gewebes und dem Anpressdruck der beweglichen Branche 112.

Um die aktuelle Resonanzfrequenz des Ultraschall-Transducers 113 zu Beginn einer Aktivierungsphase zu bestimmen, führt der Ultraschallgenerator 20 einen sogenannten Scan durch, wobei der Ultraschall-Transducer nacheinander mit mehreren Frequenzen angesteuert wird und der Verlauf der Phasenlage von Strom und Spannung des ersten hochfrequenten elektrischen Signals gemessen wird.

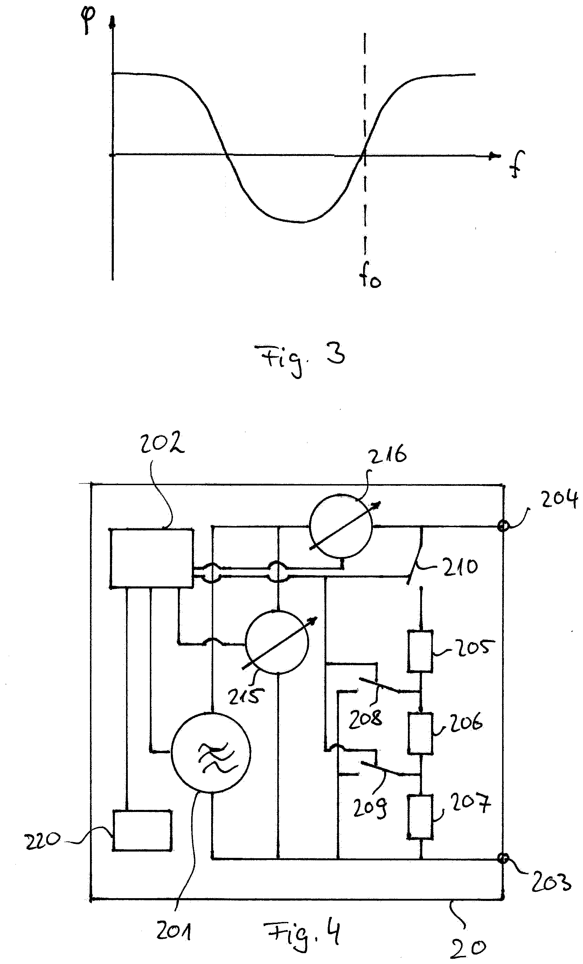

Der Verlauf der Phasenlage ϕ von Strom und Spannung in Abhängigkeit von der Frequenz f ist in Figur 3 dargestellt. Es ist zu erkennen, dass bei niedriger Frequenz zunächst eine positive Phasenlage besteht, also der Strom der Spannung voreilt. Bei steigender Frequenz sinkt die Phase ab, durchschreitet den Nullpunkt, und wird zunächst negativ. In diesem Frequenzbereich eilt die Spannung dem Strom vor. Bei Annäherung an die Resonanzfrequenz f₀ steigt die Phase ϕ wieder an und durchschreitet bei Erreichen der Resonanzfrequenz f₀ erneut den Nullpunkt, um bei weiter steigenden Frequenzen positiv zu werden. Hier eilt also wieder der Strom der Spannung vor.

Der Phasenverlauf bei niedrigen Frequenzen ist durch eine baulich bedingte Parallelkapazität bestimmt. Hierbei handelt es sich hauptsächlich um die kapazitive Wirkung von Kontaktierungsflächen, die auf den Piezokristallen des Ultraschall-Transducers aufgedampft sind.

In Figur 4 ist der Aufbau des Ultraschallgenerators 20 schematisch dargestellt, soweit er für das Verständnis der Erfindung relevant ist.

Ein Oszillator 201 erzeugt ein hochfrequentes elektrisches Signal mit steuerbarer Frequenz. Die Frequenz des Oszillators 201 wird von einer Steuerung 202 gesteuert. Das hochfrequente elektrische Signal wird an Ausgangsklemmen 203,204 bereitgestellt, welche mit Kontakten des Steckers 120 des Ultraschallinstruments 10 verbunden werden können.

Zwischen den Ausgangsklemmen 203,204 sind Induktivitäten 205,206,207 angeordnet, welche durch Schalter 208,209,210 zu- oder abgeschaltet werden können. Die Induktivitäten 205,206,207 dienen dazu, die Phasenverschiebung zwischen Strom und Spannung des hochfrequenten elektrischen Signals, welche durch die Parallelkapazität des Ultraschall-Transducers 113 verursacht ist, zu kompensieren. Die Schalter 208,209,210 werden durch die Steuerung 202 kontrolliert.

Je nach Bauart eines angeschlossenen Ultraschallinstruments werden alle oder einzelne der Induktivitäten 205,206,207 durch die Steuerung aktiviert. Die Erkennung eines angeschlossenen Ultraschallinstruments erfolgt mittels bekannter Methoden zur Instrumentenerkennung, die hier nicht näher erläutert werden müssen. Dabei können beispielsweise im Speicher 130 des Ultraschallinstruments 10 abgelegte Informationen ausgewertet werden.

Über Sensoren 215,216 werden der Strom und die Spannung des hochfrequenten elektrischen Signals in kurzen Abständen abgetastet. Aus den Abtastwerten ermittelt die Steuerung 202 die Phasenlage zwischen Strom und Spannung und regelt die Frequenz des Oszillators 201 so, dass Strom und Spannung in Phase sind, um so den Ultraschall-Transducer 113 in seiner Resonanzfrequenz anzusteuern.

Wie eingangs erläutert wurde sind sowohl die Messung der Phasenlage als auch die Kompensation der Parallelkapazität mit Ungenauigkeiten behaftet. Die Sensoren 215,216 umfassen elektronische Sensoren mit integrierten Filtern, welche selbst zu gewissen Phasenverschiebungen in den Messergebnissen führen können. Daneben schwanken die Parallelkapazitäten angeschlossener Ultraschall-Transducer 113 aufgrund von Fertigungstoleranzen. Daraus ergeben sich Fehlanpassungen, welche ihrerseits die Phasenlage zwischen Strom und Spannung des hochfrequenten elektrischen Signals verfälschen.

Um trotz der beschriebenen Ungenauigkeiten in der Phasenmessung die Resonanzfrequenz des Ultraschall-Transducers 113 während des Scan-Vorgangs genau zu bestimmen, und Betriebsfrequenz des Oszillators 201 entsprechend nachführen zu können, kann die Steuerung 202 bei der Bestimmung der Phasenlage zwischen Strom und Spannung des hochfrequenten elektrischen Signals Korrekturwerte berücksichtigen. Dazu ist in dem Ultraschallgenerator 20 ein Speicherelement 220 vorgesehen, in welchem entsprechende Korrekturwerte abgelegt werden können.

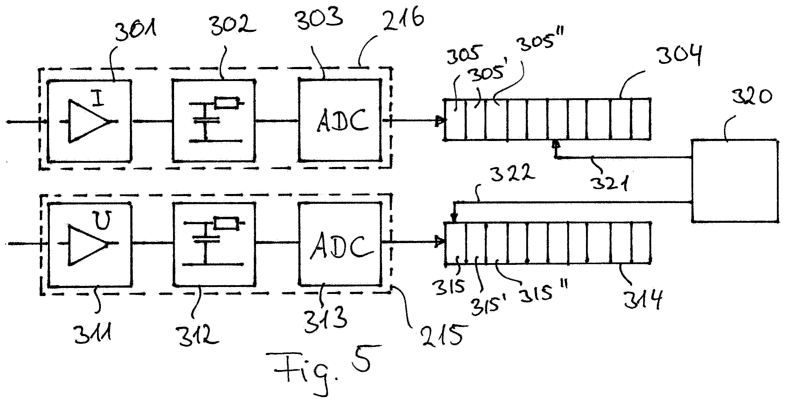

Eine Möglichkeit zur Berücksichtigung der Korrekturwerte ist in Figur 5 schematisch dargestellt. Der Sensor 216 für den Strom umfasst einen Messverstärker 301, einen Tiefpassfilter 302, und einen Analog-Digital-Wandler 303. Der Analog-Digital-Wandler 303 wandelt die Ausgangsspannung des Tiefpassfilters 302 mit einer festen Abtastrate in digitale Abtastwerte um, wobei die Grenzfrequenz des Tiefpassfilters 302 an die Abtastrate angepasst ist.

Die Abtastwerte des Analog-Digital-Wandlers 303 werden in ein Schieberegister 304 abgelegt. Das Schieberegister 304 umfasst mehrere Registerzellen 305,305',305" usw., welche jeweils einen Abtastwert aufnehmen können.

Jedes Mal, wenn ein neuer Abtastwert in das Schieberegister 304 abgelegt wird, werden dort bereits abgelegte Abtastwerte um eine Registerzelle weiter nach rechts geschoben.

In entsprechender Weise umfasst der Sensor 215 für die Spannung einen Messverstärker 311, einen Tiefpassfilter 312, und einen Analog-Digital-Wandler 313. Abtastwerte des Analog-Digital-Wandlers 313 werden in einem Schieberegister 314 mit Registerzellen 315, 315',315" usw. abgelegt. Die Arbeitsweise entspricht dabei der oben beschriebenen Arbeitsweise des Schieberegisters 304.

Eine Einheit 320 zur Bestimmung der Phasenlage zwischen Strom und Spannung greift nun auf die Inhalte der Schieberegister 304,314 zu und liest aufeinander folgende Abtastwerte für den Strom und die Spannung aus. Dabei wird anhand der in dem Speicher 220abgelegten Korrekturwerte festgelegt, an welcher Stelle der Schieberegister 304,314 miteinander korrespondierende Abtastwerte ausgelesen werden.

Muss beispielsweise eine positive Phasenverschiebung, also ein durch die weiter oben beschriebenen Effekte verursachtes Vorauseilen des Stroms, korrigiert werden, so werden die Abtastwerte des Stroms aus einer weiter rechts liegenden Registerzelle des Schieberegisters 304 ausgelesen, als die Abtastwerte der Spannung aus dem Schieberegister 314. Dies ist durch die unterschiedlich positionierten Lesezeiger 321,322 angedeutet.

Im dargestellten Beispiel wird der Abtastwert des Stroms aus der sechsten Registerzelle des Schieberegisters 304 ausgelesen, während der Abtastwert der Spannung aus der ersten Registerzelle des Schieberegisters 314 ausgelesen wird. Dadurch wird das Stromsignal vor der Ermittlung der Phasenlage um das fünffache der Abtastrate der Analog-Digital-Wandler 303,313 verzögert.

Soll hingegen ein Vorauseilen der Spannung korrigiert werden, so wird der Abtastwert des Stroms aus der ersten Registerzelle des Schieberegisters 314 ausgelesen, und der Abtastwert der Spannung wird aus einer weiter rechts liegenden Registerzelle des Schieberegisters 304 ausgelesen.

Die eigentliche Bestimmung der Phasenlage von Strom und Spannung durch die Einheit 320 kann auf verschiedene bekannte Arten erfolgen und Verfahren wie Autokorrelation, schnelle Fourier-Transformation oder ähnliches einschließen.

Die Schieberegister 304,314 können als diskrete Bauteile vorgesehen sein, und die Einheit 320 kann als digitaler Signalprozessor ausgeführt sein. Alternativ können die Schieberegister 304,314 und die Einheit 320 durch Software realisiert sein, welche in der Steuerung 202 des Ultraschallgenerators 20 ausgeführt wird. Weitere hardwarebasierte Implementierungen sind ebenfalls denkbar, beispielsweise unter Einsatz feldprogrammierbarer Gate-Arrays (FPGA)

Um die für die Phasenkompensation benötigten Korrekturwerte zu ermitteln, können die Phasengänge der Tiefpassfilter 302,312 bei einer Inbetriebnahme des Ultraschallgenerators 20 ausgemessen werden. Dazu kann beispielsweise der Ultraschallgenerator an einer rein ohmschen Last betrieben werden. Aus den Phasengängen können dann die benötigten Korrekturwerte ermittelt werden, beispielsweise in Form einer Lookup-Tabelle, in welcher für verschiedene Arbeitsfrequenzen entsprechende Verzögerungszeiten hinterlegt sind, und in dem Speicher 220 abgelegt werden.

Um eine Phasenbeeinflussung durch Fehlanpassungen zwischen den Induktivitäten 205,206,207 und der Parallelkapazität eines Ultraschall-Transducers 113 zu kompensieren, muss die Größe der Fehlanpassung bekannt sein. Dazu kann bei einem erstmaligen Verbinden eines Ultraschallinstruments 10 mit einem Ultraschallgenerator 20 die Fehlanpassung ausgemessen werden, woraus dann die Korrekturwerte bestimmt werden können.

Alternativ kann bei der Herstellung des Ultraschallinstruments 10 die Parallelkapazität des Ultraschall-Transducers 113 genau ausgemessen und in dem Speicher 130 des Ultraschallinstruments abgelegt werden. Ebenso können während der Herstellung des Ultraschallgenerators 20 die Induktivitäten 205,206,207 genau ausgemessen und in dem Speicher 220 des Ultraschallgenerators 20 abgelegt werden.

Wird nun das Ultraschallinstrument 10 mit dem Ultraschallgenerator 20 verbunden, so liest die Steuerung 202 des Ultraschallgenerators den Wert der Parallelkapazität aus dem Speicher 130, und die Werte der Induktivitäten 205,206,207 aus dem Speicher 220, und errechnet die sich aus diesen Werten ergebende Fehlanpassung sowie entsprechende Korrekturwerte. Diese Korrekturwerte können dann in der oben beschriebenen Weise zur Phasenkorrektur genutzt werden.

## Patentansprüche

1. Elektrochirurgisches System mit:
- einem Ultraschallgenerator (20), der zur Abgabe eines hochfrequenten elektrischen Signals eingerichtet ist, und
- einem Ultraschallinstrument (10), welches einen Ultraschall-Transducer (113) umfasst, der zum Umsetzen des hochfrequenten elektrischen Signals in eine Ultraschallschwingung eingerichtet ist,
wobei der Ultraschallgenerator (20) ferner eingerichtet ist, eine Resonanzfrequenz des Ultraschall-Transducers (113) zu bestimmen und eine Frequenz des hochfrequenten elektrischen Signals an diese Resonanzfrequenz anzupassen, und
wobei der Ultraschallgenerator (20) weiter eingerichtet ist, eine Phasenlage zwischen dem Strom und der Spannung des hochfrequenten elektrischen Signals zu ermitteln und anhand der ermittelten Phasenlage zu bestimmen, ob die Frequenz des hochfrequenten elektrischen Signals der Resonanzfrequenz des Ultraschall-Transducers (113) entspricht,
**dadurch gekennzeichnet, dass** der Ultraschallgenerator (20) eingerichtet ist, bei der Bestimmung der Phasenlage Korrekturwerte zu berücksichtigen, die in einem Speicher (130,220) des Ultraschallgenerators (20) und/oder des Ultraschallinstruments (10) hinterlegt sind oder hinterlegbar sind.

2. Elektrochirurgisches System nach Anspruch 1, **dadurch gekennzeichnet, dass** der Ultraschallgenerator (20) Sensoren (214,215) zur Messung des Stroms und/oder der Spannung des hochfrequenten Signals aufweist, wobei ein Frequenz- und/oder Phasengang der Sensoren (214,215) die Ermittlung der Phasenlage zwischen dem Strom und der Spannung des hochfrequenten elektrischen Signals beeinflusst, und wobei die Korrekturwerte erste Korrekturwerte umfassen, durch deren Berücksichtigung diese Beeinflussung ganz oder teilweise kompensiert wird.

3. Elektrochirurgisches System nach Anspruch 1 oder 2, **dadurch gekennzeichnet, dass** der Ultraschall-Transducer (113) eine kapazitive Last darstellt, und dass der Ultraschallgenerator (20) eine Induktivität (205,206,207) umfasst, um durch die kapazitive Last verursachte Blindströme zu unterdrücken, wobei eine Fehlanpassung zwischen der kapazitiven Last und der Induktivität (205,206,207) die Ermittlung der Phasenlage zwischen dem Strom und der Spannung des hochfrequenten elektrischen Signals beeinflusst, und wobei die Korrekturwerte zweite Korrekturwerte umfassen, durch deren Berücksichtigung diese Beeinflussung ganz oder teilweise kompensiert wird.

4. Elektrochirurgisches System nach Anspruch 3, **dadurch gekennzeichnet, dass** die zweiten Korrekturwerte in dem Ultraschallinstrument (10) gespeichert sind, und dass der Ultraschallgenerator (20) eingerichtet ist, die zweiten Korrekturwerte bei einer Verbindung des Ultraschallinstruments (10) mit dem Ultraschallgenerator (10) auszulesen.

5. Elektrochirurgisches System nach Anspruch 3 oder 4, **dadurch gekennzeichnet, dass** der Ultraschallgenerator (10) mehrere Induktivitäten (205,206,207) umfasst, welche in Abhängigkeit von einem Typ eines angeschlossenen Ultraschallinstruments (10) wahlweise zu- oder abgeschaltet werden können.

6. Elektrochirurgisches System nach Anspruch 5, **dadurch gekennzeichnet, dass** die Zu- und/oder Abschaltung der Induktivitäten (205,206,207) auf Basis einer automatischen Instrumentenerkennung erfolgt.

7. Elektrochirurgisches System nach einem der vorangehenden Ansprüche, **dadurch gekennzeichnet, dass** die ersten und/oder die zweiten Korrekturwerte durch eine Kalibrierungsmessung bestimmt oder bestimmbar sind.

8. Elektrochirurgisches System nach einem der vorangehenden Ansprüche, **dadurch gekennzeichnet, dass** der Ultraschallgenerator (20) eingerichtet ist, Strom und Spannung des hochfrequenten elektrischen Signals zeitdiskret abzutasten und aus dem Verlauf der Abtastwerte die Phasenlage zu bestimmen, wobei zur Berücksichtigung der Korrekturwerte
- die Abtastwerte des Stroms gegenüber den Abtastwerten der Spannung um eine aus den Korrekturwerten abgeleitete Zeitdifferenz verzögert werden, oder
- die Abtastwerte der Spannung gegenüber den Abtastwerten des Stroms um eine aus den Korrekturwerten abgeleitete Zeitdifferenz verzögert werden.

9. Ultraschallgenerator für ein elektrochirurgisches Systems nach einem der vorangehenden Ansprüche, wobei der Ultraschallgenerator zur Abgabe eines hochfrequenten elektrischen Signals eingerichtet ist, wobei der Ultraschallgenerator ferner eingerichtet ist, eine Resonanzfrequenz eines Ultraschall-Transducers eines Ultraschallinstruments zu bestimmen und eine Frequenz des hochfrequenten elektrischen Signals an diese Resonanzfrequenz anzupassen, und wobei der Ultraschallgenerator weiter eingerichtet ist, eine Phasenlage zwischen dem Strom und der Spannung des hochfrequenten elektrischen Signals zu ermitteln und anhand der ermittelten Phasenlage zu bestimmen, ob die Frequenz des hochfrequenten elektrischen Signals der Resonanzfrequenz des Ultraschall-Transducers entspricht, **dadurch gekennzeichnet, dass** der Ultraschallgenerator eingerichtet ist, bei der Bestimmung der Phasenlage Korrekturwerte zu berücksichtigen, die in einem Speicher des Ultraschallgenerators und/oder des Ultraschallinstruments hinterlegt sind oder hinterlegbar sind.

## Claims

1. Electrosurgical system comprising:
- an ultrasonic generator (20) configured to output a high frequency electrical signal, and
- an ultrasonic instrument (10) comprising an ultrasonic transducer (113) configured to convert the high frequency electrical signal into an ultrasonic vibration,
wherein the ultrasonic generator (20) is further configured to determine a resonant frequency of the ultrasonic transducer (113) and to adjust a frequency of the high-frequency electrical signal to this resonant frequency, and
wherein the ultrasonic generator (20) is further configured to determine a phase shift between the current and the voltage of the high-frequency electrical signal and to determine from the determined phase shift whether the frequency of the high-frequency electrical signal corresponds to the resonant frequency of the ultrasonic transducer (113),
**characterized in that** the ultrasonic generator (20) is configured to consider, when determining the phase shift, correction values which are stored or storable in a memory (130, 220) of the ultrasonic generator (20) and/or of the ultrasonic instrument (10).

2. Electrosurgical system according to claim 1, **characterized in that** the ultrasonic generator (20) comprises sensors (214, 215) for measuring the current and/or the voltage of the high-frequency signal, wherein a frequency and/or phase response of the sensors (214, 215) influences the determination of the phase shift between the current and the voltage of the high-frequency electrical signal, and wherein the correction values comprise first correction values, by consideration of which this influence is fully or partially compensated.

3. Electrosurgical system according to claim 1 or 2, **characterized in that** the ultrasonic transducer (113) constitutes a capacitive load, and **in that** the ultrasonic generator (20) comprises an inductance (205, 206, 207) to suppress reactive currents caused by the capacitive load, wherein a misadjustment between the capacitive load and the inductance (205, 206, 207) influences the determination of the phase shift between the current and the voltage of the high-frequency electrical signal, and wherein the correction values comprise second correction values, by consideration of which this influence is fully or partially compensated.

4. Electrosurgical system according to claim 3, **characterized in that** the second correction values are stored in the ultrasonic instrument (10), and **in that** the ultrasonic generator (20) is configured to read out the second correction values when the ultrasonic instrument (10) is connected to the ultrasonic generator (10).

5. Electrosurgical system according to claim 3 or 4, **characterized in that** the ultrasonic generator (10) comprises a plurality of inductances (205, 206, 207) which can be selectively switched on or off depending on a type of a connected ultrasonic instrument (10).

6. Electrosurgical system according to claim 5, **characterized in that** the inductances (205, 206, 207) are switched on and/or off on the basis of automatic instrument recognition.

7. Electrosurgical system according to any one of the preceding claims, **characterized in that** the first and/or the second correction values are determined or determinable by a calibration measurement.

8. Electrosurgical system according to any one of the preceding claims, **characterized in that** the ultrasonic generator (20) is configured to sample a discrete-time representation of the current and voltage of the high-frequency electrical signal and to determine the phase shift from the course of the sampled values, wherein, in order to consider the correction values
- the sampled values of the current are delayed relative to the sampled values of the voltage by a time difference derived from the correction values, or
- the sampled values of the voltage are delayed in relation to the sampled values of the current by a time difference derived from the correction values.

9. Ultrasonic generator for an electrosurgical system according to any one of the preceding claims, wherein the ultrasonic generator is configured to output a high-frequency electrical signal, wherein the ultrasonic generator is further configured to determine a resonant frequency of an ultrasonic transducer of an ultrasonic instrument and to adjust a frequency of the high-frequency electrical signal to this resonant frequency, and wherein the ultrasonic generator is further configured to determine a phase shift between the current and the voltage of the high-frequency electrical signal and to determine from the determined phase shift whether the frequency of the high-frequency electrical signal corresponds to the resonant frequency of the ultrasonic transducer, to determine from the determined phase shift whether the frequency of the high-frequency electrical signal corresponds to the resonant frequency of the ultrasonic transducer, **characterized in that** the ultrasonic generator is configured to consider, when determining the phase shift, correction values which are stored or storable in a memory of the ultrasonic generator and/or the ultrasonic instrument.

## Revendications

1. Système électrochirurgical comprenant :
- un générateur d'ultrasons (20) qui est configuré pour émettre un signal électrique à haute fréquence, et
- un instrument à ultrasons (10) comprenant un transducteur à ultrasons (113) adapté pour convertir le signal électrique à haute fréquence en une vibration ultrasonore, dans lequel le générateur d'ultrasons (20) est en outre configuré pour déterminer une fréquence de résonance du transducteur à ultrasons (113) et pour adapter une fréquence du signal électrique à haute fréquence à cette fréquence de résonance, et dans lequel le générateur d'ultrasons (20) est en outre configuré pour déterminer un déphasage entre le courant et la tension du signal électrique à haute fréquence et pour déterminer, à l'aide du déphasage déterminé, si la fréquence du signal électrique à haute fréquence correspond à la fréquence de résonance du transducteur à ultrasons (113),
**caractérisé en ce que** le générateur d'ultrasons (20) est configuré pour prendre en compte, lors de la détermination du déphasage, des valeurs de correction qui sont déposées ou déposables dans une mémoire (130, 220) du générateur d'ultrasons (20) et/ou de l'instrument à ultrasons (10).

2. Système électrochirurgical selon la revendication 1, **caractérisé en ce que** le générateur d'ultrasons (20) comprend des capteurs (214, 215) pour mesurer le courant et/ou la tension du signal à haute fréquence, dans lequel une réponse en fréquence et/ou en phase des capteurs (214, 215) influence la détermination du déphasage entre le courant et la tension du signal électrique à haute fréquence, et dans lequel les valeurs de correction comprennent des premières valeurs de correction dont la prise en compte permet de compenser totalement ou partiellement cette influence.

3. Système électrochirurgical selon la revendication 1 ou 2, **caractérisé en ce que** le transducteur à ultrasons (113) représente une charge capacitive, et **en ce que** le générateur d'ultrasons (20) comprend une inductance (205, 206, 207) pour supprimer les courants réactifs provoqués par la charge capacitive, dans lequel une désadaptation entre la charge capacitive et l'inductance (205, 206, 207) influence la détermination du déphasage entre le courant et la tension du signal électrique à haute fréquence, et dans lequel les valeurs de correction comprennent des deuxièmes valeurs de correction dont la prise en compte permet de compenser totalement ou partiellement cette influence.

4. Système électrochirurgical selon la revendication 3, **caractérisé en ce que** les deuxièmes valeurs de correction sont mémorisées dans l'instrument à ultrasons (10), et **en ce que** le générateur d'ultrasons (20) est configuré pour lire les deuxièmes valeurs de correction lors d'une connexion de l'instrument à ultrasons (10) au générateur d'ultrasons (10).

5. Système électrochirurgical selon la revendication 3 ou 4, **caractérisé en ce que** le générateur d'ultrasons (10) comprend plusieurs inductances (205, 206, 207) qui peuvent être sélectivement activées ou désactivées en fonction d'un type d'instrument à ultrasons (10) raccordé.

6. Système électrochirurgical selon la revendication 5, **caractérisé en ce que** l'activation et/ou la désactivation des inductances (205, 206, 207) s'effectue sur la base d'une reconnaissance automatique de l'instrument.

7. Système électrochirurgical selon l'une des revendications précédentes, **caractérisé en ce que** les premières et/ou les deuxièmes valeurs de correction sont déterminées ou déterminables par une mesure d'étalonnage.

8. Système électrochirurgical selon l'une des revendications précédentes, **caractérisé en ce que** le générateur d'ultrasons (20) est configuré pour échantillonner le courant et la tension du signal électrique à haute fréquence de manière discrète dans le temps et pour déterminer le déphasage à partir de l'évolution des valeurs d'échantillonnage, dans lequel, pour tenir compte des valeurs de correction
- les valeurs d'échantillonnage du courant sont retardées par rapport aux valeurs d'échantillonnage de la tension d'une différence de temps déduite des valeurs de correction, ou
- les valeurs d'échantillonnage de la tension sont retardées par rapport aux valeurs d'échantillonnage du courant d'une différence de temps déduite des valeurs de correction.

9. Générateur d'ultrasons pour un système électrochirurgical selon l'une des revendications précédentes, le générateur d'ultrasons étant configuré pour émettre un signal électrique à haute fréquence, dans lequel le générateur d'ultrasons est en outre configuré pour déterminer une fréquence de résonance d'un transducteur à ultrasons d'un instrument à ultrasons et pour adapter une fréquence du signal électrique à haute fréquence à cette fréquence de résonance, et dans lequel le générateur d'ultrasons est en outre configuré pour déterminer un déphasage entre le courant et la tension du signal électrique à haute fréquence et pour déterminer, à l'aide du déphasage déterminé, si la fréquence du signal électrique à haute fréquence correspond à la fréquence de résonance du transducteur à ultrasons, **caractérisé en ce que** le générateur d'ultrasons est configuré pour prendre en compte, lors de la détermination du déphasage, des valeurs de correction qui sont déposées ou déposables dans une mémoire du générateur d'ultrasons et/ou de l'instrument à ultrasons.
